# EUROPÄISCHE PATENTSCHRIFT

(11) **EP 0 742 025 B1**
(45) Veröffentlichungstag und Bekanntmachung des Hinweises auf die Patenterteilung: **17.10.2001**
(21) Anmeldenummer: 96107129.7
(22) Anmeldetag: 07.05.1996
(51) Int. Cl.: A61M 5/142, A61M 5/155

(54) **Verfahren zum Befüllen einer Pumpenkammer einer gasdruckbetriebenen Medikamentenpumpe**
Pump chamber filling procedure for a pressurized gas-driven drug pump
Procédé pour remplir une chambre d'une pompe pour médicaments entraînée par gaz à pression

(30) Priorität: 11.05.1995 DE 19517291
(43) Veröffentlichungstag der Anmeldung: 13.11.1996
(73) Patentinhaber: Fresenius AG, 61350 Bad Homburg v.d.H (DE)
(72) Erfinder: Langkau, Wolfram, 61449 Steinbach (DE)
(74) Vertreter: Luderschmidt, Schüler & Partner GbR

(56) Entgegenhaltungen:
- EP-A- 0 142 866
- US-A- 5 197 322

## Beschreibung

Die vorliegende Erfindung betrifft ein Verfahren zum Befüllen einer gasdruckbetriebenen Medikamentenpumpe mit einem fluiden Treibmittel nach dem Oberbegriff des Patentanspruchs 1. Sie betrifft insbesondere ein Verfahren zum Befüllen einer implantierbaren Infusionspumpe mit Treibmittel.

Gasdruckbetriebene Medikamentenpumpen, insbesondere implantierbare Infusionspumpen werden zur kontinuierlichen Medikation (gleichbleibende Dosis) über längere Zeiträume bei Patienten eingesetzt, die sonst nur durch Injizieren der Medikamente, wie Morphinen, Heparinen usw., mehrmals täglich behandelt werden können. Die Pumpen haben gegenüber der üblichen Injektion den Vorteil, daß die verabreichte Dosis nicht mehr so weit überdosiert werden muß, daß trotz des Abbaus des Medikamentes bis zum nächsten Verabreichungszeitpunkt eine gewisse Mindestdosis nicht unterschritten wird, sondern eine gleichmäßige und insgesamt wesentlich verringerte Zufuhr des Medikamentes verwirklicht werden kann.

Eine derartige Medikamentenpumpe weist eine Pumpenkammer auf, die durch eine flexible Trennwand, z.B. eine Membran oder ein Faltenbalg, in einen das Medikament enthaltenden Raum und einen Treibmittel enthaltenden Raum unterteilt ist. Die Wirkungsweise einer derartigen Medikamentenpumpe ist derart, daß das in der Pumpe enthaltene Treibmittel im Betriebszustand im Gleichgewicht zwischen flüssiger und gasförmiger Phase vorliegt. Der Dampfdruck des Treibmittels muß bei der Betriebstemperatur ausreichend über dem Atmosphärendruck liegen, so daß über die flexible Trennwand stetig Druck auf den Medikamentenraum ausgeübt werden kann, so daß diese das Medikament über ein Reduziersystem gegen den Umgebungsdruck in den Körper des Patienten abgibt. Die Volumenvergrößerung des Treibgasraumes wird von einer weiteren Verdampfung des Treibmittels aufgefangen und geschieht so lange isobar, solange das Treibmittel als 2-Phasensystem vorliegt.

Derartige Medikamentenpumpen sind beispielsweise aus DE 43 33 736 und EP 0 075 062 bekannt. DE 43 33 736 beschreibt eine Pumpe, deren Medikamentenraum durch einen Balg gebildet wird. Die Außenseite des Balges liegt in einer druckdichten Dose, in der sich ein Treibmittel befindet, das einen konstanten Druck erzeugt, durch den der Balg zusammengedrückt und das Medikament ausgetrieben wird. Demgegenüber beschreibt die EP 0 075 062 eine Pumpe, in der der Treibmittelraum durch eine undurchlässige Membran von der Wirkstoffkammer abgetrennt ist. Der Druck, der im Treibmittelraum durch das Treibmittel erzeugt wird, wird über die Membran auf die Wirkstoffkammer ausgeübt, so daß der Wirkstoff dosiert abgegeben wird.

Für die Befüllung eines Treibmittelraumes mit Treibmittel sind unterschiedliche Verfahren bekannt. In der EP 0 142 866 ist ein Verfahren zum Befüllen einer implantierbaren Infusionspumpe offenbart. Diese Infusionspumpe besteht aus zwei Teilen, die erst nach dem Einfüllen des Treibmittels miteinander verbunden werden. Der Treibmittelraum ist auf der einen Seite von dem unteren Teilgehäuse, auf der anderen Seite von einem Diaphragma begrenzt. Für die Treibmittelbefüllung wird zunächst eine Treibmittelmischung hergestellt, die bei Körpertemperatur einen Dampfdruck von 0,55 bis 0,62 bar hat. Die Mischung wird anschließend unter Vakuum von der gelösten Luft befreit. Daraufhin werden die Mischung und der Treibmittelraum auf 51,7°C erhitzt. Nachdem Vakuum an den Treibmittelraum und die äußere Diaphragmaseite angelegt worden ist, wird das erwärmte Treibmittel durch eine kleine Öffnung in dem unteren Teilgehäuse in den Treibmittelraum und den an die äußere Diaphragmaseite angrenzenden Raum eingeführt. Anschließend wird mit Luft oder Stickstoff Druck auf die äußere Diaphragmaseite ausgeübt, so daß ein Großteil des Treibmittels wieder aus dem Treibmittelraum ausgestoßen wird. Die Öffnung in dem unteren Teilgehäuse wird mit einem Stopfen verschlossen und das untere Teilgehäuse mit dem oberen verbunden. Dieses Verfahren hat jedoch den großen Nachteil, daß es sehr aufwendig ist und erheblich größere Mengen Treibmittel für die Befüllung benötigt werden, als für den Betrieb der Pumpe später nötig sind.

Ein anderes Verfahren, das in der DE-A 195 09 632 offenbart ist, benötigt zur Treibmittelbefüllung einer implantierbaren Infusionspumpe dagegen nur die Menge Treibmittel, die zum Betrieb notwendig ist. Der Treibmittelraum dieser Pumpe wird von zwei tiefgezogenen Metallfolien oder Metallverbundfolien gebildet, die im Randbereich des gesamten Umfangs durch Randverklebung miteinander verbunden sind. In dieses pillenförmige Gebilde wird durch die Dichtfläche eine Mikrokapillare eingeführt, durch die die Kammer zunächst evakuiert und anschließend mit Treibmittel gefüllt wird. Die Nachteile dieses Verfahrens bestehen darin, daß die Kapillaren sehr empfindlich sind und leicht brechen, wodurch die Pumpe unbrauchbar wird. Ferner ist die Dichtfläche zwischen den Folien nicht ausreichend, so daß Fremdgase über die Dichtfläche in den Treibmittelraum eindringen können.

Durch das Eindringen von Fremdgasen in die Pumpenkammer ist jedoch eine reibungslose Funktion einer Infusionspumpe nicht mehr gewährleistet, da die Verdampfung des Treibmittels nicht mehr unter isobaren Bedingungen erfolgt. Der Druck, der auf die Membran wirkt, verringert sich mit der Volumenzunahme des Treibmittelraumes, und damit verringert sich auch die Förderrate des Medikamentes.

Aufgabe der Erfindung ist es daher, ein Verfahren bereitzustellen, mit dem der Treibmittelraum einer Pumpenkammer einer gasdruckbetriebenen Pumpe, insbeondere eine implantierbare Infusionspumpe, einfach gefüllt werden kann, ohne daß Fremdgase in die Pumpenkammer eindringen können.

Erfindungsgemäß wird die Aufgabe durch ein Verfahren gemäß dem kennzeichnenden Teil des Anspruchs 1 gelöst.

Der Treibmittelraum wird erfindungsgemäß mit Treibmittel gefüllt, indem ein mit Treibmittel gefülltes Kissen in den Treibmittelraum der Pumpenkammer gelegt wird und dieser evakuiert wird. Das Kissen besteht vorteilhaft aus einem Material, durch das das Treibmittel hindurchdiffundieren kann, das von dem Treibmittel jedoch nicht angegriffen wird. Anschließend wird der Treibmittelraum gasdicht verschlossen. Durch die Wand des Treibmittelkissens diffundiert das Treibmittel langsam im nachfolgenden nach außen. Unter langsam ist der Zustand zu verstehen, daß nach ca. 15 Minuten das Treibmittel durch die Mikroporen des Polymermaterials nach außen diffundiert ist. In dem Treibmittelraum bleibt das leere Treibmittelkissen zurück. Es ist aber auch möglich, ein im wesentlichen für das Treibmittel undurchlässiges Material zu verwenden, das durch Anlegen von Unterdruck von außen her zum Platzen gebracht wird.

In einer bevorzugten Ausführungsform wird der Treibmittelraum der Pumpenkammer aus zwei tiefgezogenen Verbundfolien, einer Metall- oder Metallverbundfolie und einer gasundurchlässigen Trennwand gebildet, wie es in der DE-A 195 09 634 offenbart ist. Das Treibmittelkissen wird zwischen die beiden Folien gelegt und der Raum zwischen den beiden Folien evakuiert. Die ein Außenkissen bildenden Folien können zusammen mit dem Treibmittelkissen in die Pumpenkammer eingesetzt werden, so daß das Außenkissen den Treibmittelraum begrenzt. Es ist aber auch möglich, die Pumpenkammer durch eine flexible Trennwand in den Wirkstoffraum und den Treibmittelraum zu unterteilen und das pillenförmige Gebilde aus tiefgezogenen Verbundfolien zusammen mit dem Treibmittelkissen in den Raum der Pumpenkammer einzulegen, der von der flexiblen Trennwand und der Gehäusewandung der Pumpenkammer begrenzt wird. Vorzugsweise wird auf das pillenförmige Gebilde eine die Trennwand bildende Folie aufgebracht, die am Pumpenkammerrand befestigt wird. Die Folie kann zwischen den beiden Gehäuseschalen der Pumpenkanner verklemmt und/oder verklebt werden.

Bevorzugt werden die tiefgezogenen Folien zunächst entlang ihres Außenrandes miteinander verbunden, wobei am Rand zwischen den Folien eine Evakuierungsöffnung verbleibt. Durch diese Öffnung wird ein Rohr eingebracht, durch das der Raum zwischen den beiden Folien evakuiert wird. Anschließend wird die Öffnung verschlossen.

Eine besonders bevorzugte Ausführungsform der Erfindung sieht vor, daß die beiden Folien durch Randverschweißung miteinander verbunden werden. Die Randverschweißung erfolgt vorzugsweise nach dem Ultraschall-Schweißverfahren. Dieses Schweißverfahren ist für die Erfindung insbesondere deshalb geeignet, weil es sehr kurze Schweißzeiten von weniger als einer Sekunde ermöglicht. Durch dieses Verfahren wird die Dichtwirkung der Verbundfolien am Umfang erheblich verbessert.

Zwischen Kissenbefüllung und Treibmittelraum-Verschweißung sollten nicht mehr als zwei Minuten vergehen, so daß in der Zwischenzeit nur eine geringe Menge des Treibmittels austreten kann.

Geeigneterweise besteht das Kissen aus dem Element einer Luftpolsterfolie, auf die ein Silikonplättchen aufgeklebt ist. Durch das Silikonplättchen wird das Kissen mit Hilfe zweier Nadeln evakuiert und befüllt.

Die eingefüllte Treibmittelmenge ist gering und kann z.B. 2 ml betragen. Das Innenvolumen der Druckkammer kann z.B. 40 ml ausmachen.

Als Treibmittel können erfindungsgemäß alle üblichen Treibmittel, wie sie in Medikamentenpumpen verwendet werden, eingesetzt werden. Vorzugsweise wird bei implantierbaren Infusionspumpen als Treibmittel Hexafluorbutan verwendet, wobei 1,1,1,4,4,4-Hexafluorbutan besonders bevorzugt wird. Implantierbare Infusionspumpen, die diese Treibmittel enthalten, sind in der Patentanmeldung DE-A 195 09 632 beschrieben.

Mit dem oben beschriebenen Verfahren kann die Gasdichtigkeit des Treibmittelraumes während des Befüllungsvorgangs erheblich verbessert werden. Ein bedeutender Vorteil dieses Verfahrens ist ferner, daß keine Mikrokapillaren mehr direkt in den Treibmittelraum eingeführt werden müssen, womit die Gefahr, daß sie abbrechen und die Pumpe infolgedessen unbrauchbar wird, ausgeschlossen ist. Die Ausschußrate bei der Pumpenherstellung ist damit wesentlich reduziert, wodurch sich sowohl eine Erhöhung der Produktqualität als auch eine Verminderung der kumulierten Herstellungskosten ergibt.

Im folgenden werden zwei Ausführungsbeispiele der Erfindung unter Bezugnahme auf die Zeichnungen näher erläutert.

Es zeigen:
- Fig. 1: einen Schnitt durch eine inplantierbare Medikamentenpumpe in schematischer Darstellung, deren Pumpenkammer durch eine flexible Trennwand in einen Medikamentenraum und einen Treibmittelraum unterteilt ist, wobei der Treibmittelraum ein mit Treibmittel befülltes Kissen enthält, das für das Treibmittel durchlässig ist, und
- Fig. 2: einen Schnitt durch eine implantierbare Medikamentenpumpe in schematischer Darstellung, in deren Pumpenkammer ein Außenkissen aus zwei miteinander verschweißten Folien eingesetzt ist, das ein Treibmittelkissen enthält.

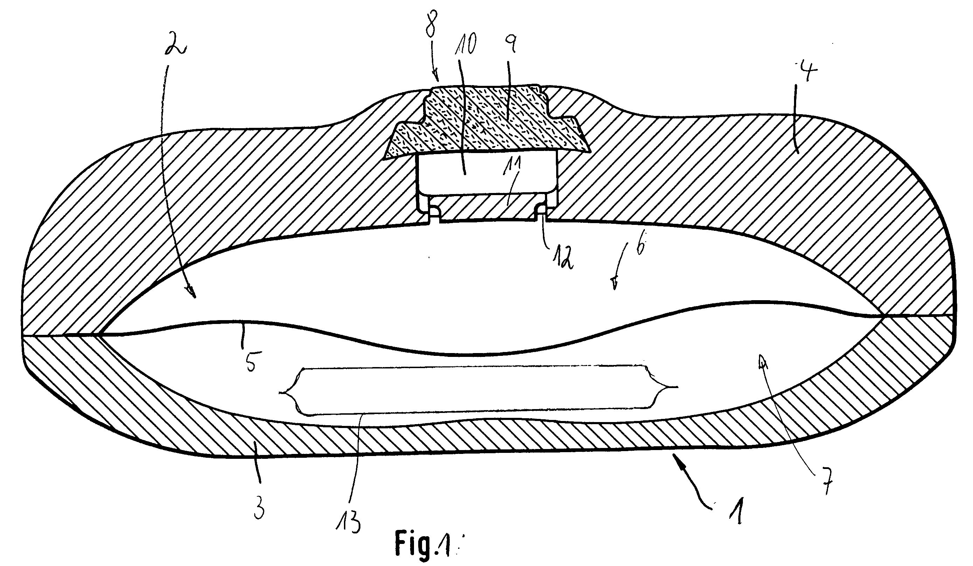

Fig. 1 zeigt einen Schnitt durch eine erste Ausführungsform einer implantierbaren Infusionspumpe in schematischer Darstellung. Die Infusionspumpe weist ein scheibenförmiges, rotationssymmetrisches Gehäuse 1 aus Hartkunststoff mit einer Pumpenkammer 2 auf, das aus einem schalenförmigen Kammerunterteil 3 und einem schalenförmigen Kammeroberteil 4 besteht. Die Pumpenkammer 2 ist durch eine flexible, für das Treilbmittel undurchlässige Trennwand 5 in einen Medikamentenraum 6 zur Aufnahme eines Medikamentes und einen Treibmittelraum 7 zur Aufnahme des als Treibmittel eingesetzten 1,1,1,4,4,4-Hexafluorbutan getrennt. Die Trennwand 5 ist mit ihrem äußeren Randbereich zwischen den Randbereichen des Kammerunterteils 3 und des Kammeroberteils 4 verklemmt bzw. eingepreßt. Das Innenvolumen der Treibmittelkammer 7 beträgt etwa 40 ml, der Medikamentenraum 6 kann ca. 30 ml der medizinischen Lösung aufnehmen. Die Infusionspumpe ist zur kontinuierlichen dosierten Abgabe von Medikamenten, wie z.B. Heparin, künstlichem Pankreasinsulin, Chemotherapeutika, Schmerzmittel etc. geeignet. Im Zentrum des Kammeroberteils 4 ist eine Nachfüllöffnung 8 vorgesehen, die durch ein Zentralseptum 9 dicht verschlossen ist. Unter dem Zentralseptum 9 befindet sich ein Nachfüllraum 10 mit einer festen Platte 11 als Nadelstopp und Durchtrittsöffnungen 12 zum Medikamentenraum 6.

Der Treibmittelraum 7 wird wie folgt mit Treibmittel befüllt. Zunächst wird aus einer Luftpolsterfolie, d.h. eine Folie, die eine Vielzahl von luftgefüllten Kammern enthält, ein Element ausgestanzt. Auf das Element wird ein Silikonplättchen mit einer Dicke von etwa 1 mm und einer Abmessung von 6 x 9 mm mit Sekundenkleber aufgeklebt. Mittels zweier Nadeln mit einem Außendurchmesser von 0,4 mm wird das Kissen evakuiert und anschließend wieder mit Treibmittel befüllt. Vor dem Zusammenbau der beiden Gehäusehälften 3, 4 der Infusionspumpe wird das in Fig. 1 mit dem Bezugszeichen 13 versehene Kissen in das Kammerunterteil 3 eingelegt. Anschließend werden Kammerunterteil 3 und Kammeroberteil miteinander verbunden, wobei die flexible Trennwand 5 zwischen den beiden Gehäusehälften verklemmt wird. Das in dem Kissen enthaltene Treibgas diffundiert dann innerhalb von etwa 15 Minuten über die dünne Wandung des Kissens in den Treibmittelraum.

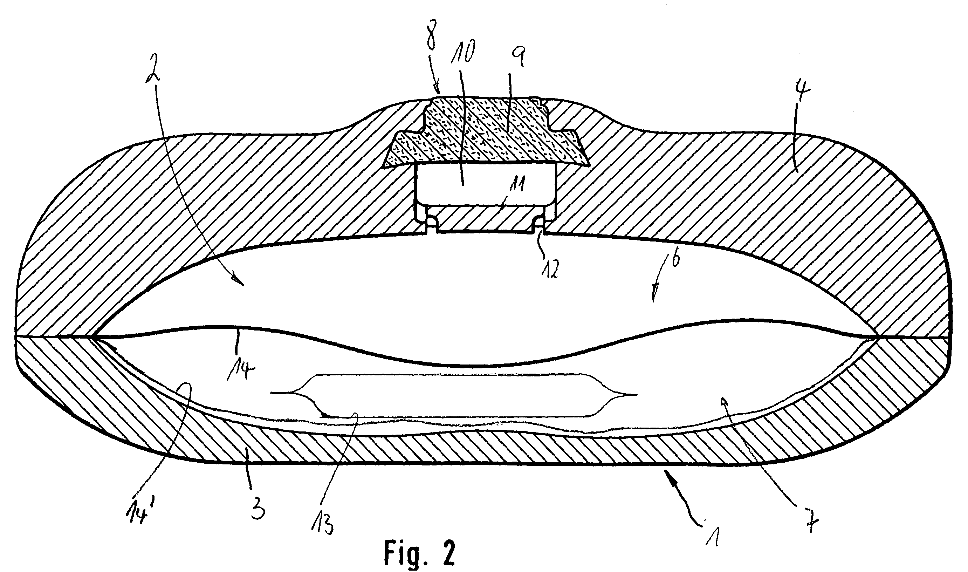

Fig. 2 zeigt eine weitere Ausführungsform der implantierbaren Infusionspumpe in schematischer Darstellung, wobei die Teile, die den Teilen der Pumpe gemäß Fig.1 entsprechen, mit den gleichen Bezugszeichen versehen sind. Die Infusionspumpe weist ein scheibenförmiges, rotationssymmetrisches Gehäuse 1 aus Hartkunststoff mit einer Pumpenkammer 2 auf, das aus einem schalenförmigen Kammerunterteil 3 und einem schalenförmigen Kammeroberteil 4 besteht. Im Zentrum des Kammeroberteils ist eine Nachfüllöffnung 8 vorgesehen, die durch ein Zentralseptum 9 dicht verschlossen ist. Unter dem Zentralseptum 9 befindet sich ein Nachfüllraum 10 mit einer festen Platte 11 als Nadelstopp und Durchtrittsöffnung 12 zur Pumpenkammer 2. Bei dieser Ausführungsform wird der Treibmittelraum 7 durch zwei tiefgezogene Folien 14, 14' gebildet, die miteinander verschweißt sind. In dem pillenförmigen Treibmittelraum 7 befindet sich das mit Treibmittel befüllte Kissen 13. Der Treibmittelraum wird wie folgt mit Treibmittel befüllt. Zunächst wird das Kissen 13 hergestellt. Daraufhin wird das Kissen zwischen den tiefgezogenen Folien, die den pillenförmigen Treibmittelraum bilden, gelegt. Anschließend werden die Folien entlang ihres Außenrandes verschweißt, wobei am Rand zwischen den Folien eine Evakuierungsöffnung verbleibt. Durch diese Öffnung wird ein Rohr eingeführt, durch das der Raum zwischen den beiden Folien evakuiert wird. Mit Hilfe einer Ultraschall-Schweißmaschine und einer geeigneten Elektrode werden die Folien anschließend verschweißt. Zwischen Kissenbefüllung und Verschweißung sollten nicht mehr als zwei Minuten vergehen. Die das Kissen enthaltenden Folien werden dann in das schalenförmige Kammerunterteil 3 eingelegt und die beiden Gehäusehälften werden miteinander verbunden, wobei die Folien an ihrem Rand verklemmt werden. Das in dem Kissen 13 enthaltene Treibmittel diffundiert nun in den pillenförmigen Treibmittelraum 7. Ferner ist es möglich, auf die miteinander verschweißten Folien, eine weitere Folie aufzubringen, die eine flexible Trennwand bildet. Diese Trennwand kann zusammen mit dem Kissen zwischen den beiden Gehäusehälften der Infusionspumpe verklemmt werden.

## Patentansprüche

1. Verfahren zum Befüllen eines Treibmittelraumes einer gasdruckbetriebenen Pumpe, insbesondere einer implantierbaren Infusionspumpe, mit einem fluiden Treibmittel, bei dem die Pumpenkammer (2) der gasdruckbetriebenen Pumpe durch eine flexible, für des Treibmittel undurchlässige Trennwand (5) in den Treibmittelraum (7) und einen Flüssigkeitsspeicherraum (6) unterteilt, der Treibmittelraum im wesentlichen von Gasen befreit und mit dem fluiden Treibmittel gefüllt wird, **dadurch gekennzeichnet, daß** ein für das Treibmittel im wesentlichen durchlässiges, jedoch inertes Kissen (13) mit dem Treibmittel gefüllt, das Kissen in den Treibmittelraum (7) eingelegt und der Treibmittelraum gasdicht verschlossen wird.

2. Verfahren nach Anspruch 1, **dadurch gekennzeichnet, daß** der Treibmittelraum nach dem Einlegen des mit dem Treibmittel gefüllten Kissens (13) evakuiert wird.

3. Verfahren nach Anspruch 1 oder 2, **dadurch gekennzeichnet, daß** das mit Treibmittel gefüllte Kissen (13) zwischen zwei tiefgezogene für das Treibmittel undurchlässige Folien (14,14') gelegt, die Folien an ihren Rändern unter Bildung eines eine Evakuierungsöffnung aufweisenden Außenkissens miteinander verbunden, die im Außenkissen enthaltende Luft abgezogen, die Evakuierungsöffnung verschlossen und das einen pillenförmigen Treibmittelraum bildende Außenkissen in die Pumpenkammer eingelegt wird, wobei eine der Folien (14, 14') die flexible Trennwand (5) bildet.

4. Verfahren nach Anspruch 3, **dadurch gekennzeichnet, daß** mindestens eine weitere Folie auf das Außenkissen aufgelegt und die mindestens eine Folie zusammen mit dem Außenkissen am Rand der Pumpenkammer befestigt wird.

5. Verfahren nach einem der Ansprüche 1 bis 4, **dadurch gekennzeichnet, daß** das mit Treibmittel gefüllte Kissen aus einem Element einer Luftpolsterfolie hergestellt wird.

6. Verfahren nach Anspruch 5, **dadurch gekennzeichnet, daß** ein Element einer Luftpolsterfolie ausgestanzt, auf das das Kissen bildende Element der Luftpolsterfolie ein Silikonplättchen aufgebracht und das Kissen mittels zweier Nadeln durch das Silikonplättchen evakuiert und zugleich mit dem Treibmittel befüllt wird.

7. Verfahren nach einem der Ansprüche 1 bis 6, **dadurch gekennzeichnet, daß** zwischen dem Befüllen des Kissens mit Treibmittel und dem Verschließen des Treibmittelraumes nicht mehr als zwei Minuten vergehen.

8. Gasdruckbetriebene Pumpe, insbesondere implantierbare Infusionspumpe, mit einer Pumpenkammer (2), die durch eine flexible, für das Treibmittel undurchlässige Trennwand (5) in einen Flüssigkeitsspeicherraum (6) und einen Treibmittelraum (7) unterteilt ist, **dadurch gekennzeichnet, daß** in den Treibmittelraum (7) ein mit Treibmittel befülltes Kissen (13) eingelegt ist, das für das Treibmittel durchlässig, jedoch gegenüber dem Treibmittel inert ist.

9. Gasduckbetriebene Pumpe, insbesondere implantierbare Infusionspumpe, nach Anspruch 8, **dadurch gekennzeichnet, daß** der Treibmittelraum (7) durch ein Außenkissen aus tiefgezogenen Verbundfolien (14,14') gebildet ist, in dem das mit Treibmittel gefüllte Kissen angeordnet ist.

## Claims

1. A method of filling a propellant space of a gas pressure-operated pump, particularly of an implantable infusion pump, with a fluid propellant, in which the pump chamber (2) of the gas pressure-operated pump is sub-divided by a flexible separating wall (5) which is impervious to the propellant into the propellant space (7) and a fluid storage space (6), the propellant space is substantially freed from gases and is filled with the fluid propellant, **characterised in that** a cushion (13) which is substantially permeable to the propellant but which is however inert, is filled with the propellant, the cushion is placed in the propellant space (7) and the propellant space is closed in a gas-tight fashion.

2. A method according to claim 1, **characterised in that** the propellant space is evacuated after the propellant-filled cushion (13) has been placed in it.

3. A method according to claim 1 or 2, **characterised in that** the propellant-filled cushion (13) is placed between two deep-drawn films (14, 14') which are impervious to the propellant, the films are connected to each other around their edges to form an outer cushion which has an evacuating orifice, the air contained in the outer cushion is extracted, the evacuating orifice is sealed and the outer cushion which forms a pellet-shaped propellant space, is placed into the pump chamber, one of the films (14, 14') constitutes the flexible separating wall (5).

4. A method according to claim 3, **characterised in that** at least one further film is placed on the outer cushion and the at least one film, together with the outer cushion, is fixed to the edge of the pump chamber.

5. A method according to one of claims 1 to 4, **characterised in that** the propellant-filled cushion is produced from an element of an air cushion film.

6. A method according to claim 5, **characterised in that** an element of an air cushion film is stamped out, a silicone disc is applied to the element of the air cushion film which forms the cushion and the cushion is evacuated by means of two needles through the silicone disc and is at the same time filled with a propellant.

7. A method according to one of claims 1 to 6, **characterised in that** no more than two minutes pass between filling the cushion with propellant and sealing the propellant space.

8. A gas pressure-operated pump, particularly an implantable infusion pump with a pump chamber (2) which is separated by a flexible separating wall (5) which is impervious to the propellant into a fluid storage space (6) and a propellant space (7), **characterised in that** a propellant-filled cushion (13) which is permeable to the propellant but inert in respect of the propellant, is placed into the propellant space (7).

9. A gas pressure-operated pump, particularly an implantable infusion pump, according to claim 8, **characterised in that** the propellant space (7) is constituted by an outer cushion of deep-drawn bonded films (14, 14') in which the propellant-filled cushion is disposed.

## Revendications

1. Procédé de remplissage d'une pompe actionnée par pression gazeuse, en particulier une pompe de perfusion implantable, avec un fluide propulseur, dans lequel la chambre (2) de la pompe actionnée par pression gazeuse est divisée par une cloison (5), flexible et imperméable au gaz propulseur, en un compartiment à gaz propulseur (7) et un compartiment à solution médicamenteuse (6), les gaz contenus dans le compartiment à gaz propulseur sont sensiblement évacués hors de celui-ci, que l'on remplit avec le fluide propulseur, **caractérisé en ce qu'**un coussin (13) sensiblement perméable au gaz propulseur, néanmoins inerte, est rempli avec le gaz propulseur, le coussin est posé dans le compartiment à gaz propulseur (7) et le compartiment à gaz propulseur (7) est ensuite fermé de manière étanche aux gaz.

2. Procédé selon la revendication 1, **caractérisé en ce que**, après la pose du coussin (13) rempli de gaz propulseur, le vide est produit à l'intérieur du compartiment à gaz propulseur.

3. Procédé selon la revendication 1 ou 2, **caractérisé en ce que** le coussin (13) rempli de gaz propulseur est posé entre deux feuilles (14, 14') thermoformées, imperméables au gaz propulseur, les feuilles sont soudées l'une contre l'autre sur leurs bords en formant un coussin externe muni d'un orifice d'évacuation, l'air contenu dans le coussin externe est aspiré, l'orifice d'évacuation est obturé et le coussin externe formant un compartiment à gaz propulseur en forme de pastille est posé dans la chambre de la pompe, l'une des feuilles (14, 14') formant la cloison (5) flexible.

4. Procédé selon la revendication 3, **caractérisé en ce qu'**au moins une autre feuille est posée sur le coussin externe et ladite feuille, au moins au nombre d'une, est fixée avec le coussin externe contre le bord de la chambre de la pompe.

5. Procédé selon l'une quelconque des revendications 1 à 4, **caractérisé en ce que** le coussin rempli de gaz propulseur est formé par un élément provenant d'un film à bulles d'air.

6. Procédé selon la revendication 5, **caractérisé en ce que** l'on découpe un élément dans un film à bulles d'air, on pose une plaquette de silicone sur l'élément découpé dans le film à bulles d'air et formant le coussin, puis, au moyen de deux trocarts qui passent à travers la plaquette de silicone, on évacue l'air contenu dans le coussin et, en même temps, on introduit le gaz propulseur dans le coussin.

7. Procédé selon l'une quelconque des revendications 1 à 6 **caractérisé en ce que** le temps compris entre le processus de remplissage du coussin et le processus de soudage du compartiment à gaz propulseur ne dépasse pas deux minutes.

8. Pompe actionnée par pression gazeuse, en particulier pompe de perfusion implantable, comprenant une chambre (2), qui est divisée par une cloison (5), flexible et imperméable au gaz propulseur, en un compartiment à gaz propulseur (7) et un compartiment à solution médicamenteuse (6), **caractérisée en ce que** dans le compartiment à gaz propulseur (7) est posé un coussin (13) rempli de gaz propulseur, qui est perméable au gaz propulseur, mais inerte face au gaz propulseur.

9. Pompe actionnée par pression gazeuse, en particulier pompe de perfusion implantable, selon la revendication 8, **caractérisée en ce que** le compartiment à gaz propulseur (7) est formé par un coussin externe réalisé dans des feuilles multicouches (14, 14') thermoformées, dans lequel est disposé le coussin contenant le gaz propulseur.
